# EUROPÄISCHE PATENTANMELDUNG

(11) **EP 2 266 458 A1**
(43) Veröffentlichungstag der Anmeldung: **29.12.2010**
(21) Anmeldenummer: 09008183.7
(22) Anmeldetag: 23.06.2009
(51) Int. Cl.: A61B 5/00

(54) **Sensor für in-vivo Messungen**

(71) Anmelder: Roche Diagnostics GmbH, 68305 Mannheim (DE); F. Hoffmann-La Roche AG, 4070 Basel (CH)
(72) Erfinder: Kube, Oliver, 67549 Worms (DE); Rittinghaus, Andrea, 69239 Neckarsteinach (DE)
(74) Vertreter: Mommer, Niels

(57) **Zusammenfassung**

Die Erfindung geht aus von einem Sensor für in-vivo Messungen, mit einem Substrat (1), das plan ist und einen mehrere Elektroden (2) tragenden Sensorschaft (1b) sowie einen Sensorkopf (1a) zum Anschließen des Sensors bildet, wobei der Sensorkopf (1a) zur Kontaktgabe metallisierte Flächen als Kontaktfelder (4) trägt, die über Leiterbahnen (3) mit den Elektroden (2) verbunden sind. Erfindungsgemäß ist vorgesehen, dass der Sensorkopf (1a) seitlich von dem Sensorschaft (1b) absteht, so dass die Kontaktfelder (4) seitlich von dem Sensorschaft (1 b) angeordnet sind und die Leiterbahnen (3) auf dem Sensorkopf (1a) nebeneinander quer zur Längsrichtung des Sensorschafts (1 b) verlaufen. Die Erfindung betrifft ferner ein System mit einem solchen Sensor und einem dazu passenden Steckverbinder.

## Beschreibung

Die Erfindung geht aus von einem Sensor für in-vivo Messungen mit den im Oberbegriff des Anspruchs 1 angegebenen Merkmalen. Ein derartiger Sensor ist aus der US 5,390,671 bekannt.

Derartige Sensoren ermöglichen eine elektrochemische Messung von Analyten in einem menschlichen oder tierischen Körper, beispielsweise von Glucose oder Lactat. Dazu wird ein auf einem als Sensorschaft ausgebildeten distalen Bereich eines Substrats angeordnetes Elektrodensystem in den Körper eines Patienten eingebracht, so dass elektrochemisch eine transkutane Messung durchgeführt werden kann. Dieser Vorgang wird häufig als Insertion des Sensors bezeichnet. In der Regel wird der Sensorschaft mit einer geschlitzten Kanüle in den Körper eines Patienten eingestochen, die nach der Insertion aus dem Körper des Patienten herausgezogen werden kann, so dass der Sensorschaft mit den Elektroden im Körper des Patienten stecken bleibt. Ein proximaler Bereich des Substrats ragt nach der Insertion aus dem Körper heraus und bildet einen Sensorkopf zum Anschließen des Sensors an ein Messgerät. Der Sensorkopf trägt metallisierte Flächen mit Kontaktfeldern und ist meist als ein Steckteil ausgebildet, so dass zum Anschließen des Sensors an ein Mess- oder Auswertegerät eine Kupplung oder Buchse mit einem passenden Schlitz auf das Steckteil aufgesteckt werden kann. Die Anordnung der Kontaktfelder gibt dabei die Steckrichtung vor, in der ein Steckverbinder zum Anschließen des Sensors auf den Sensorkopf aufgesteckt wird.

Wie alle medizinischen Geräte müssen Sensoren zur in-vivo Messung höchsten Anforderungen an die Zuverlässigkeit genügen. Darüber hinaus sollen die Sensoren möglichst leicht handhabbar sein, so dass sie idealerweise auch von medizinischen Laien verwendet werden können. Ferner soll der Gebrauch eines Sensors für Patienten mit möglichst geringen Schmerzen verbunden sein.

Aufgabe der Erfindung ist es, einen Weg aufzuzeigen, wie diese Anforderungen besser erfüllt werden können.

Diese Aufgabe wird durch einen Sensor mit den im Anspruch 1 angegebenen Merkmalen sowie durch ein System gemäß Anspruch 15 gelöst. Vorteilhafte Weiterbildungen der Erfindungen sind Gegenstand von Unteransprüchen.

Bei einem erfindungsgemäßen Sensor steht der Sensorkopf seitlich von dem Sensorschaft ab, so dass die Kontaktfelder seitlich von dem Sensorschaft angeordnet sind. Die durch die Anordnung der Kontaktfelder vorgegebene Steckrichtung ist vorteilhaft quer zur Längsrichtung des Sensorschafts.

Zum Anschließen des Sensors an ein Mess- oder Auswertegerät kann deshalb eine Steckverbindung durch eine quer zu einem von der Kanüle erzeugten Einstichkanal und entlang der Hautoberfläche eines Patienten verlaufende Steckbewegung geschlossen werden. Beispielsweise kann ein Kupplungsteil, das einen zu dem Sensorkopf passenden Schlitz hat, auf den Sensorkopf aufgesteckt werden. Möglich ist es aber auch, dass der Sensorkopf in einem Stützteil angeordnet ist, das zusammen mit dem Sensorkopf einen elektrischen Steckverbinder ausbildet.

Bei einem erfindungsgemäßen Sensor ist deshalb keine Kraft in Längsrichtung des Einstichkanals erforderlich, um diesen an ein Mess- oder Auswertegerät anzuschließen. Demzufolge kann die Gefahr vermieden werden, dass die Kanüle oder der Sensor durch eine zum Schließen einer Steckverbindung erforderliche Kraft unbeabsichtigt noch tiefer in den Körper eines Patienten gedrückt und somit unnötige Schmerzen verursacht werden. Vorteilhaft ist deshalb der Gebrauch eines erfindungsgemäßen Sensors für einen Patienten mit weniger Schmerzen verbunden.

Zudem wird das Anschließen eines erfindungsgemäßen Sensors an ein Messgerät von Patienten auch aus psychologischen Gründen als angenehmer empfunden. Das Ausüben einer Kraft in Stichrichtung eines im eigenen Körper steckenden Sensors wird instinktiv als bedrohlich und deshalb unangenehm empfunden. Mit einem erfindungsgemäßen Sensor kann dieses Problem vermieden werden. Dies führt dazu, dass Patienten, die den Sensor selbstständig an ein Messgerät anschließen, dabei psychologisch nicht gehemmt sind und deshalb eine Steckverbindung leichter fehlerfrei vornehmen können. Ein erfindungsgemäßer Sensor hat deshalb auch den Vorteil, Messergebnisse mit einer erhöhten Zuverlässigkeit zu liefern.

Ein wichtiger Vorteil eines erfindungsgemäßen Sensors ist insbesondere auch, dass man das Substrat zum Anschließen des Sensors an ein Messgerät nicht zu biegen braucht. Indem die Kontaktierung des Sensors von Biegekräften entlastet wird, kann auch Zuverlässigkeit der Steckverbindung erhöht und der Aufbau des das Steckteil aufnehmenden Kupplungsteils vereinfacht werden.

Das Substrat eines erfindungsgemäßen Sensors kann biegsam ausgebildet sein, beispielsweise indem es durch Ausschneiden aus einer Kunststofffolie hergestellt wird. Ein biegsames Substrat hat den Vorteil, dass sich der Sensor im Körper eines Patienten an dessen Bewegungen anpassen kann. Die Angabe, dass das Substrat eines erfindungsgemäßen Sensors flach ist, bezieht sich deshalb auf einen neuen, ungebrauchten Sensor, auf dessen Substrat keine Biegekräfte einwirken. In entsprechende Weise bezieht sich auch die Angabe, dass das Steckteil eine Steckrichtung vorgibt, die mit der Längsrichtung der Kanüle einen spitzen Winkel einschließt, auf einen neuen, ungebrauchten Sensor im kräftefreien Zustand.

Der Sensorkopf eines erfindungsgemäßen Sensors kann als ein Steckteil ausgebildet sein. Zum Anschließen des Sensors an ein Messgerät kann der Sensorkopf dann von einem Benutzer in einen Schlitz einer Buchse oder eines Kupplers gesteckt und auf diese Weise eine Steckverbindung geschlossen werden. Der Sensorkopf kann aber auch von einem Hersteller mit einem Steckteil verbunden werden, das von einem Benutzer zum Anschließen des Sensors mit einem dazu passenden Steckverbindungsteil zusammengesteckt wird.

Bei einem ungebrauchten Sensor ist das Substrat bevorzugt hochkant in dem Schlitz einer Kanüle angeordnet. Mit der Kanüle wird im Körper eines Patienten ein Einstichkanal erzeugt. Die die Kanüle kann nach einem Einstich entfernt werden, wobei das Substrat in dem Einstichkanal stecken bleibt. Nach der Insertion ist die von dem bevorzugt als Steckteil ausgebildeten Sensorkopf vorgegebene Steckrichtung deshalb im kräftefreien Zustand quer zu dem Einstichkanal.

Bei einem erfindungsgemäßen Sensor wird vorteilhaft ein flaches Substrat verwendet, das mit geringem Aufwand beispielsweise dadurch hergestellt werden, dass es aus einem Kunststoffblatt ausgeschnitten wird. In einer Seitenansicht, also mit Blickrichtung senkrecht auf die Schmalseiten des Substrats, hat das Substrat dann die Form eines geradlinigen Streifens. Ein von dem Substrat als Sensorkopf gebildetes Steckteil kann kostengünstig nach dem Prinzip einer Steckkarte ausgebildet werden. Dazu können auf dem Steckteil bzw. dem das Steckteil bildenden Abschnitt des Substrates zur Kontaktgabe metallisierte Flächen als Kontaktfelder angeordnet werden, die über Leiterbahnen jeweils mit einer der Elektroden verbunden sind.

Die Elektroden und Kontaktfelder können ähnlich wie bei einer Leiterplatte auf einer Oberseite oder Unterseite des Substrats angeordnet sein. Dabei können sich alle Elektroden und Kontaktfelder auf einer einzigen Seite des Substrats befinden, die dann üblicherweise als Oberseite bezeichnet wird. Möglich ist es aber auch, dass sowohl auf der Oberseite als auf der Unterseite Elektroden und/oder Kontaktfelder angeordnet sind. Bevorzugt ist dabei ein Kontaktfeld auf der Oberseite elektrisch leitend mit einem Kontaktfeld auf der Unterseite verbunden, um die Kontaktierung zu verbessern. Möglich ist es aber auch, dass die Kontaktfelder auf der Oberseite und auf der Unterseite jeweils mit unterschiedlichen Elektroden verbunden sind, so dass sich durch Ausnutzung von Ober- und Unterseite ein besonders kompaktes Design verwirklichen lässt.

Der Sensorkopf eines erfindungsgemäßen Sensors ist bevorzugt länglich ausgebildet und die Längsrichtung des Sensorkopfs quer zur Längsrichtung des Sensorschafts. Bevorzugt ist ferner, dass der Sensorkopf breiter als der Sensorschaft ist.

Der Sensorkopf kann sich senkrecht zur Längsrichtung des Sensorschafts erstrecken, so dass die Leiterbahnen auf dem Sensorkopf senkrecht zur Längsrichtung des Sensorschafts verlaufen. Auf diese Weise lässt sich ein Steckteil bilden, das eine senkrecht zur Längsrichtung des Sensorschafts verlaufende Steckrichtung vorgibt. Allerdings werden Sensoren für in vivo Messungen in der Regel nicht senkrecht zur Hautoberfläche in den Körper eines Patienten, sondern schräg dazu eingestochen. Bevorzugt erstreckt sich der Sensorkopf deshalb schräg zur Längsrichtung des Sensorschafts und gibt eine Steckrichtung vor, die schräg zur Längsrichtung des Sensorschafts verläuft. Hierfür ist es vorteilhaft, wenn die Leiterbahnen auf dem Sensorkopf schräg zur Längsrichtung des Sensorschafts verlaufen. Wenn der Sensor schräg zur Hautoberfläche in den Körper eines Patienten eingestochen wird, kann die Steckverbindung dann nämlich ergonomisch vorteilhaft geschlossen werden, indem ein Steckverbinder zum Kontaktieren des Sensorkopfes näherungsweise parallel zur Hautoberfläche bewegt wird.

Weitere Einzelheiten und Vorteile der Erfindung werden an einem Ausführungsbeispiel unter Bezugnahme auf die beigefügten Zeichnungen erläutert. Gleiche und einander entsprechende Komponenten sind dabei mit übereinstimmenden Bezugszahlen bezeichnet. Es zeigen:
- Figur 1: einen erfindungsgemäßen Sensor;
- Figur 2: den in Figur 1 dargestellten Sensor ohne Kanüle;
- Figur 3: eine Seitenansicht zu Figur 2;
- Figur 4: eine Querschnittsansicht zu Figur 1;
- Figur 5: eine Ansicht der Kanülenspitze;
- Figur 6: ein weiteres Ausführungsbeispiel eines erfindungsgemäßen Sensors;
- Figur 7: das in Figur 2 gezeigte Ausführungsbeispiel mit einem Stützteil;
- Figur 8: eine Schnittansicht zu Figur 7;
- Figur 9: einen Steckverbinder zu dem in Figur 7 gezeigten Ausführungsbeispiel;
- Figur 10: das in Figur 7 gezeigte Ausführungsbeispiel zusammen mit einer Trä- gereinheit.

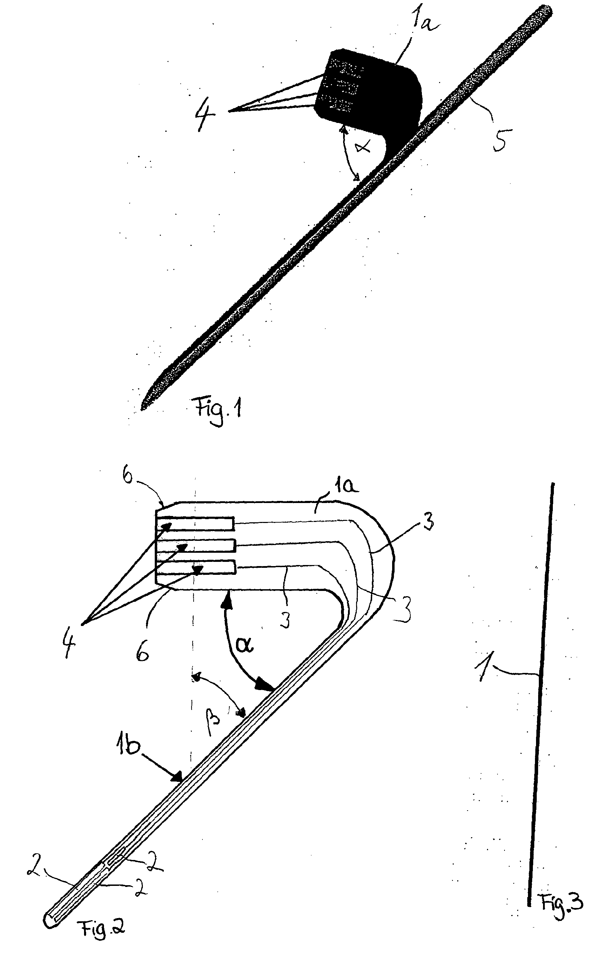

Der in den Figuren 1 bis 4 dargestellte Sensor für in-vivo Messungen hat ein flaches Substrat 1, das einen Sensorkopf 1 a und einen von dem Sensorkopf 1 a ausgehenden Sensorschaft 1 b bildet. Der Sensorschaft 1 b trägt Elektroden 2 für elektrochemische Messungen, die jeweils über Leiterbahnen 3 mit auf dem Sensorkopf 1 a angeordneten Kontaktfeldern 4 verbunden sind.

Das Substrat 1 ist plan. Der Sensorschaft 1b und der Sensorkopf 1 a haben deshalb eine coplanare Oberfläche, genauer gesagt zwei coplanare Oberflächen, nämlich die Oberseite und die Unterseite des Substrats 1, da das Substrat 1 aus einem Kunststoffblatt ausgeschnitten wurde. In einer Seitenansicht, also mit Blickrichtung senkrecht auf die Schmalseiten des Sensors, erscheint der Sensor deshalb als geradliniger Streifen, wie die in Figur 3 dargestellt ist.

Der Sensorkopf 1 a steht seitlich von dem Sensorschaft 1b ab, so dass die Kontaktfelder 4 seitlich von dem Sensorschaft 1b angeordnet sind und die Leiterbahnen 3 auf dem Sensorkopf 1a nebeneinander quer zur Längsrichtung des Sensorschafts 1 b verlaufen.

Zum Anschließen des Sensors an ein Messgerät kann der Sensorkopf 1 a mit den Kontaktfeldern 4 in eine passende Buchse gesteckt werden. Vorteilhaft erfolgt die dabei erforderliche Steckbewegung wegen der Anordnung der Kontaktfelder 4 quer zur Längsrichtung des Sensorschafts 1 a. Eine solche Bewegung lässt sich für einen Benutzer leicht durchführen. Dabei lässt sich insbesondere die Gefahr vermeiden, dass der Sensor tiefer in den Körper hineingedrückt und dabei unnötig Schmerzen verursacht werden.

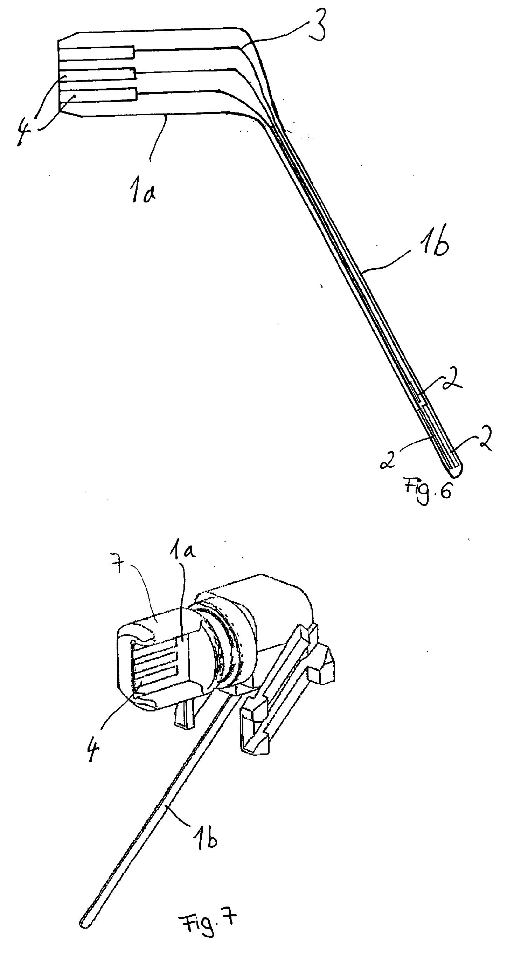

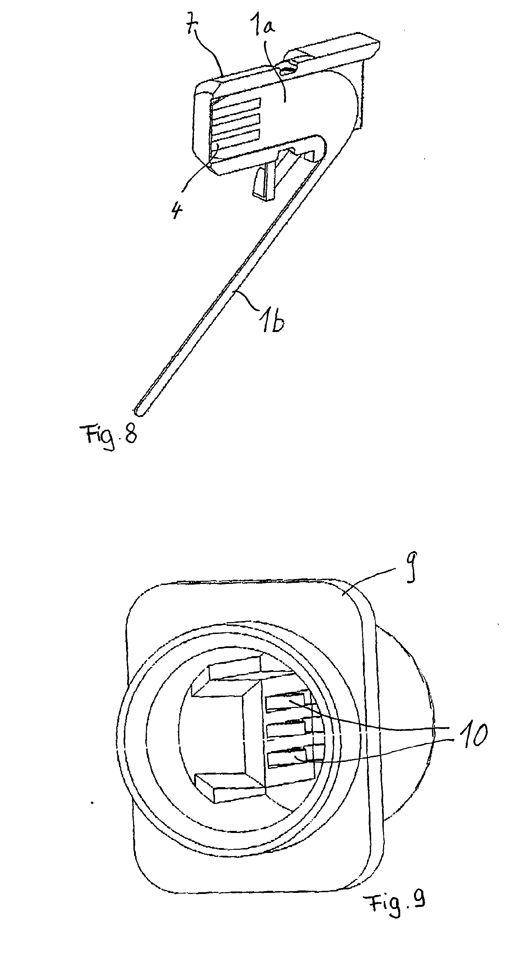

Bei einem dünnen und deshalb flexiblen Substrat kann das Aufstecken einer Buchse auf den Sensorkopf 1a manchen Benutzern Schwierigkeiten bereiten. Um dem abzuhelfen kann der Sensorkopf 1a vom Hersteller in einem Stützteil angeordnet werden, das zusammen mit dem Sensorkopf 1a einen elektrischen Steckverbinder ausbildet, dessen Verbindungsrichtung, also die Steckrichtung, in der ein dazu passender Steckverbinder zum Aufstecken bewegt werden muss, quer zur Längsrichtung des Sensorschaftes 1b verläuft. Ein Ausführungsbeispiel eines Sensors mit einem solchen Stützteil 7 ist in Figur 7 in einer Seitenansicht und in Figur 8 in einer Schnittansicht dargestellt. In Figur 9 ist zusätzlich ein Steckverbinder 9 dargestellt, der zu dem von dem Stützteil 7 und dem Sensorkopf 1 a gebildeten Steckverbinder passt und zusammen mit dem Sensor ein System bildet. Der Steckverbinder 9 hat federnd bewegliche Linienkontakte 10, die sich in der Steckrichtung erstrecken und beim Schließen der Steckverbindung jeweils eines der Kontaktfelder 4 des Sensors kontaktieren. Der Sensorkopf 1 a kann für sich alleine oder zusammen mit einem Stützteil 7 einen Steckverbinder bilden.

Ob mit oder ohne Stützteil kann der Sensorkopf 1a also durch eine Steckverbindung elektrisch an ein Messgerät angeschlossen werden. Die Kontaktfelder sind dabei derart auf dem Sensorkopf angeordnet, dass ein Steckverbinder zum Anschließen des Sensors an ein Messgerät nur durch eine quer zur Längsrichtung verlaufende Steckbewegung auf den Sensorschaft 1 b aufgesteckt werden kann.

Die durch die Anordnung der Kontaktfelder 4 auf dem Sensorkopf 1 a vorgegebene Steckrichtung schließt mit der Längsrichtung des Sensorschafts 1 b einen Winkel α ein. Die Kontaktfelder 4 sind nebeneinander in einer Reihe auf dem Steckteil 1 a angeordnet. Die Steckrichtung ist quer zur Richtung der Reihe. Die Reihe der Kontaktfelder 4 schließt mit der Längsrichtung des streifenförmigen Sensorschafts 1b einen Winkel β ein, der in Figur 2 eingezeichnet ist.

Der Winkel α weicht bei dem dargestellten Ausführungsbeispiel um 20° bis 70°, insbesondere bei 30° bis 60°, von einem rechten Winkel ab, beträgt also im Falle eines spitzen Winkels 20° bis 70°, insbesondere 30° bis 60°, oder im Falle eines stumpfen Winkels 110° bis 160°, insbesondere 120° bis 150°. Bei dem dargestellten Ausführungsbeispiel ist der Winkel α ein spitzer Winkel. Der Sensorkopf 1 a kann jedoch auch auf der gegenüberliegenden Seite von dem Sensorschaft 1 b abstehen, wie dies in Figur 6 dargestellt ist. In einem solchen Fall ist der Winkel α ein stumpfer Winkel. Der Winkel β weicht bei den dargestellten Ausführungsbeispielen ebenfalls um 20° bis 70°, beispielsweise 30° bis 60°, von einem rechten Winkel ab.

Der Sensorkopf 1a hat seitliche Einführschrägen 6, die einem Benutzer das Aufstecken eines nicht dargestellten Kupplungsteils oder bei der Herstellung das Aufstecken eines in den Figuren 7 und 8 dargestellten Stützteils erleichtern. Diese Einführschrägen 6 sind an gegenüberliegenden Seitenrändern des Sensorkopfes 1a angeordnet. Die Breite des Sensorkopfes 1a nimmt wegen der Einführschrägen 6 zu dem vorderen Ende, auf welches zum Anschließen des Sensors ein Steckverbinder aufgesteckt wird, hin ab.

Das Substrat 1 kann ähnlich wie eine Leiterplatte mit Metall beschichtet werden, um Leiterbahnen 3 und Kontaktfelder 4 auszubilden. Die Kontaktfelder 4 sind bevorzugt länglich ausgebildet und erstrecken sich quer zur Längsrichtung des Sensorschafts 1b, bei dem dargestellten Ausführungsbeispiel ist die Längsrichtung der Kontaktfelder deshalb zugleich auch die Steckrichtung. Vorteilhaft lässt sich so die Zuverlässigkeit eines über die Kontaktfelder 4 hergestellten elektrischen Kontakts erhöhen.

Bei dem dargestellten Ausführungsbeispiel sind eine Arbeitselektrode, eine Gegenelektrode und eine Referenzelektrode vorhanden, wobei eine Referenzelektrode nicht zwingend erforderlich ist. Diese Elektroden 2 sind bei dem dargestellten Ausführungsbeispiel auf derselben Substratseite angeordnet. Prinzipiell ist es jedoch auch möglich, eine oder mehrere Elektroden auf der gegenüberliegenden Substratseite, also auf dessen Rückseite, anzuordnen, beispielsweise um ein besonders kompaktes Design zu verwirklichen. Die Rückseite des Substrats 1 lässt sich aber beispielsweise auch dazu nutzen, dort weitere Elektroden anzuordnen. Davon abgesehen können aber auch auf einer Seite des Substrats 1 mehrere Arbeitselektroden angeordnet werden. Auf diese Weise kann ein Sensor geschaffen werden, der an unterschiedliche Konzentrationsbereiche angepasste Messsensitivitäten hat oder zur Messung unterschiedlicher Analyten verwendet werden kann.

Die Elektroden 2 sind in den Figuren schematisch rechteckig dargestellt, können aber jede beliebige Gestalt haben.

Das Substrat 1 kann kostengünstig aus Kunststoff hergestellt werden, beispielsweise aus einem Blatt ausgeschnitten wird. Das Substrat 1 ist deshalb plan und hat einen rechteckigen Querschnitt, wie dies in der Seitenansicht der Figur 3 zu sehen ist. Bei dem dargestellten Ausführungsbeispiel sind der Sensorkopf 1a und der Sensorschaft 1 b gleich dick. Es ist aber auch möglich den Sensorkopf 1 a und/oder einen an den Sensorkopf 1a anschließenden Teil des Sensorschafts 1b etwas dicker auszubilden als den die Elektroden 2 tragenden Teil des Sensorschafts. Auf diese Weise kann die Stabilität des Sensors erhöht werden, so dass sich eine Steckverbindung auch ohne ein den Sensorkopf 1 a umgebendes Stützteil 7 leichter schließen lässt.

Der Sensorkopf 1 a kann vorteilhaft länglich ausgebildet sein, um das Aufstecken eines Kupplungsteils zum Anschließen des Sensors an ein Mess- oder Auswertegerät oder eines Stützteils 7 zu erleichtern. Der Sensorkopf 1 a schließt bei den gezeigten Ausführungsbeispielen mit dem geradlinig verlaufenden Sensorschaft 1b den Winkel α ein.

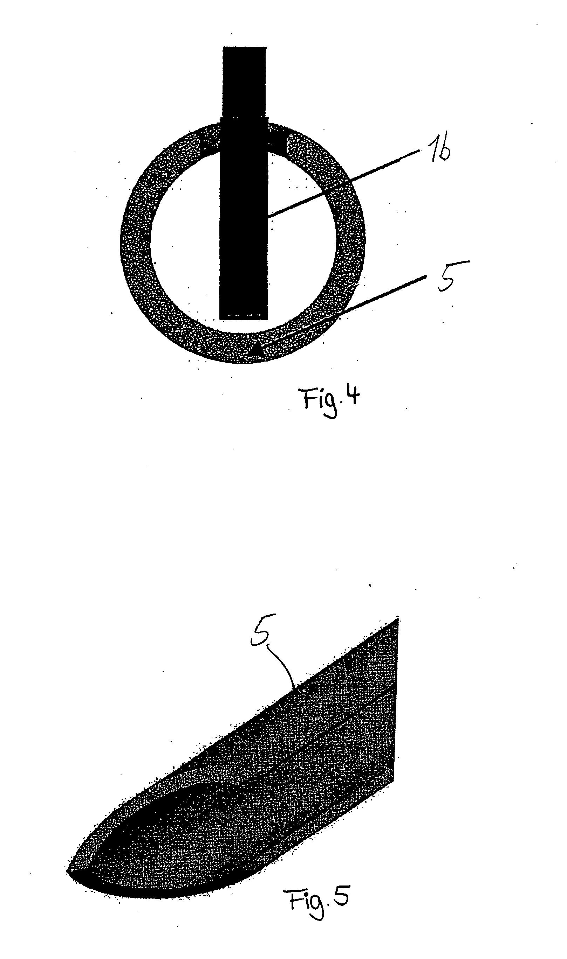

Zu dem Sensor gehört eine Kanüle 5, mit der das auf dem Sensorschaft 1 b angeordnete Elektrodensystem für transkutane Messungen in den Körper eines Patienten insertiert werden kann. Die Kanüle 5 hat einen in ihrer Längsrichtung verlaufenden Schlitz, in dem der Sensorschaft 1b hochkant angeordnet ist. Wie insbesondere Figur 4 zeigt, hat der Sensorschaft 1 b bei dem dargestellten Ausführungsbeispiel eine Breite, die größer als der Innendurchmesser der Kanüle 5 ist, so dass der Sensorschaft 1b aus dem Schlitz herausragt. Es ist aber auch möglich, dass die Breite des Sensorschafts 1 b kleiner als der Kanülendurchmesser ist.

Da das Substrat 1 in dem Schlitz hochkant angeordnet ist, ist eine Schmalseite des Substrats 1 der dem Schlitz im inneren der Kanüle 5 gegenüberliegenden Innenfläche der Kanüle zugewandt. Die gegenüberliegende Schmalseite des Substrats 1, genauer gesagt des als Streifen ausgebildeten Sensorschafts 1b, ist von der Kanüle 5 angewandt.

Figur 5 zeigt das distale Ende der Kanüle 5. Wie darin dargestellt, hat die Kanüle 5 ein schräg angeschnittenes Ende, das in einer Spitze mündet. Der Schlitz ist dabei auf der Seite der Kanüle angeordnet, welche der die Spitze aufweisenden Seite gegen überliegt.

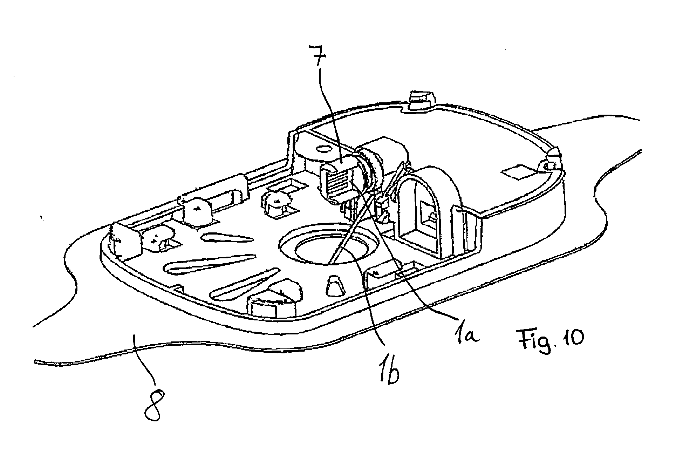

Der Sensor kann bei Gebrauch an einer Trägereinheit 8 befestigt sein, die auf den Körper eines Patienten aufgeklebt ist. Ein Beispiel einer solchen Trägereinheit 8 ist in Figur 10 dargestellt. Auf einer solchen Trägereinheit kann vorteilhaft auch eine Messeinheit befestigt werden, die den Sensor über eine Steckverbindung mit Strom versorgt.

### Bezugszahlen

- 1: Substrat
- 1 a: Sensorkopf
- 1 b: Sensorschaft
- 2: Elektrode
- 3: Leiterbahn
- 4: Kontaktfeld
- 5: Kanüle
- 6: Einführschräge
- 7: Stützteil
- 8: Trägereinheit
- 9: Steckverbinder
- 10: Linienkontakte

## Patentansprüche

1. Sensor für in-vivo Messungen, mit
einem Substrat (1), das plan ist und einen mehrere Elektroden (2) tragenden Sensorschaft (1b) sowie einen Sensorkopf (1a) bildet, der zum Anschließen des Sensors mit einem elektrischen Steckverbinder (9) kontaktiert wird und zur Kontaktgabe metallisierte Flächen als Kontaktfelder (4) trägt, die über Leiterbahnen (3) mit den Elektroden (2) verbunden sind und deren Anordnung eine Steckrichtung zum Aufstecken des Steckverbinders auf den Sensorkopf (1a) bestimmt,
**dadurch gekennzeichnet, dass**
der Sensorkopf (1a) seitlich von dem Sensorschaft (1b) absteht, so dass die Kontaktfelder (4) seitlich von dem Sensorschaft (1b) angeordnet sind und die durch die Anordnung der Kontaktfelder (4) bestimmte Steckrichtung quer zur Längsrichtung des Sensorschafts (1 b) verläuft.

2. Sensor nach Anspruch 1, **dadurch gekennzeichnet, dass** die Leiterbahnen (3) auf dem Sensorkopf (1a) nebeneinander quer zur Längsrichtung des Sensorschafts (1 b) verlaufen.

3. Sensor nach einem der vorstehenden Ansprüche, **dadurch gekennzeichnet, dass** der Sensorkopf (1 a) Einführschrägen (6) aufweist.

4. Sensor nach einem der vorstehenden Ansprüche, **dadurch gekennzeichnet, dass** der Sensorkopf (1a) mit der Längsrichtung des Sensorschafts (1 b) einen Winkel (α) einschließt, der um 20° bis 70°, bevorzugt um 30° bis 60°, von einem rechten Winkel abweicht.

5. Sensor nach einem der vorstehenden Ansprüche, **dadurch gekennzeichnet, dass** der Sensorkopf (1a) mit der Längsrichtung des Sensorschafts (1b) einen spitzen Winkel (α) einschließt

6. Sensor nach einem der vorstehenden Ansprüche, **dadurch gekennzeichnet, dass** die Kontaktfelder (4) länglich ausgebildet sind und sich quer zur Längsrichtung des Sensorschafts (1 b) erstrecken.

7. Sensor nach einem der vorstehenden Ansprüche, **dadurch gekennzeichnet, dass** die Kontaktfelder (4) nebeneinander in einer Reihe angeordnet sind, die mit der Längsrichtung des Sensorschafts (1 b) einen Winkel (β) einschließt, der um 20° bis 70°, bevorzugt um 30° bis 60°, von einem rechten Winkel abweicht.

8. Sensor nach einem der vorstehenden Ansprüche, **dadurch gekennzeichnet, dass** der Sensorschaft (1 b) ein Streifen ist, dessen Breite größer als die Dicke des Substrats (1) ist.

9. Sensor nach einem der vorstehenden Ansprüche, **dadurch gekennzeichnet, dass** der Sensorkopf (1a) länglich ist und seine Längsrichtung quer zu dem Sensorschaft (1 b) orientiert ist.

10. Sensor nach einem der vorstehenden Ansprüche, **dadurch gekennzeichnet, dass** das Substrat (1) aus einem Kunststoffblatt ausgeschnitten ist.

11. Sensor nach einem der vorstehenden Ansprüche, **dadurch gekennzeichnet, dass** die Elektroden (2) auf einer Ober- und/oder einer Unterseite des Substrats (1) angeordnet sind.

12. Sensor nach einem der vorstehenden Ansprüche, **dadurch gekennzeichnet, dass** das Substrat in einer geschlitzten Kanüle steckt.

13. Sensor nach Anspruch 11, **dadurch gekennzeichnet, dass** die Kanüle (5) ein schräg angeschnittenes Ende aufweist, das in einer Spitze mündet, wobei der Schlitz auf der Seite der Kanüle (5) angeordnet ist, welche der die Spitze aufweisenden Seite gegen überliegt.

14. Sensor nach einem der vorstehenden Ansprüche, **dadurch gekennzeichnet, dass** der Sensorkopf (1a) in einem Stützteil (7) angeordnet ist, das zusammen mit dem Sensorkopf (1a) einen elektrischen Steckverbinder (9) ausbildet.

15. System mit einem Sensor nach einem der vorstehenden Ansprüche und einem Steckverbinder (9), der zum Anschließen des Sensors auf den Sensorkopf (1a) aufgesteckt wird und dabei eine elektrische Steckverbindung schließt, **dadurch gekennzeichnet, dass** der Steckverbinder (9) nur durch eine quer zur Längsrichtung des Sensorschafts (1b) verlaufende Steckbewegung auf den Sensorkopf (1a) aufgesteckt werden kann.
